# EUROPÄISCHE PATENTSCHRIFT

(11) **EP 0 765 860 B1**
(45) Veröffentlichungstag und Bekanntmachung des Hinweises auf die Patenterteilung: **06.05.1999**
(21) Anmeldenummer: 96115455.6
(22) Anmeldetag: 26.09.1996
(51) Int. Cl.: C07C 67/327, C07C 69/54, C07C 67/08, C07C 67/60

(54) **Verfahren zum Verestern von (Meth)acrylsäure mit einem Alkanol**
Process for esterifying (meth)acrylic acid with an alcanol
Procédé d'estérification de l'acide (méth)acrylique avec un alcanol

(30) Priorität: 28.09.1995 DE 19536184
(43) Veröffentlichungstag der Anmeldung: 02.04.1997
(73) Patentinhaber: BASF AKTIENGESELLSCHAFT, 67056 Ludwigshafen (DE)
(72) Erfinder: Aichinger, Heinrich, Dr., 68199 Mannheim (DE); Fried, Michael, Dr., 69121 Heidelberg (DE); Nestler, Gerhard, Dr., 67061 Ludwigshafen (DE)
(74) Vertreter: Isenbruck, Günter, Dr.

(56) Entgegenhaltungen:
- DE-A- 2 339 519
- DE-A-19 547 485

## Beschreibung

Die Erfindung betrifft ein Verfahren zum Verestern von (Meth)acrylsäure mit einem Alkanol in Gegenwart eines Veresterungskatalysators, bei dem nicht umgesetzte Ausgangsverbindungen und der zu bildende (Meth)acrylsäureester destillativ abgetrennt werden und ein Oxyester enthaltendes Sumpfprodukt entsteht. Der Begriff (Meth)acrylsäure bezeichnet dabei in bekannter Weise Acryl- oder Methacrylsäure.

Die Herstellung von Alkylestern der (Meth)acrylsäure erfolgt üblicherweise durch Verestern von (Meth)acrylsäure mit Alkanolen bei erhöhter Temperatur in flüssiger Phase mit oder ohne Lösungsmittel und in Gegenwart von Säure als Katalysator (DE-A 23 39 519). Nachteilig an dieser Herstellungsweise ist, daß sich unter den vorgenannten Veresterungsbedingungen als Nebenreaktionen noch nicht umgesetzter Ausgangsalkohol unter Ausbildung einer Verbindung der nachfolgend angeführten allgemeinen Formel I sowie noch nicht umgesetzte (Meth)acrylsäure unter Ausbildung einer Verbindung der allgemeinen Formel II an die Doppelbindung von bereits gebildetem (Meth)acrylsäurealkylester addiert (Michael-Addition). Auch eine Mehrfachaddition ist möglich. Ferner können gemischte Typen auftreten. Diese Addukte (Alkoxyester und Acyloxyester) nennt man kurz Oxyester. 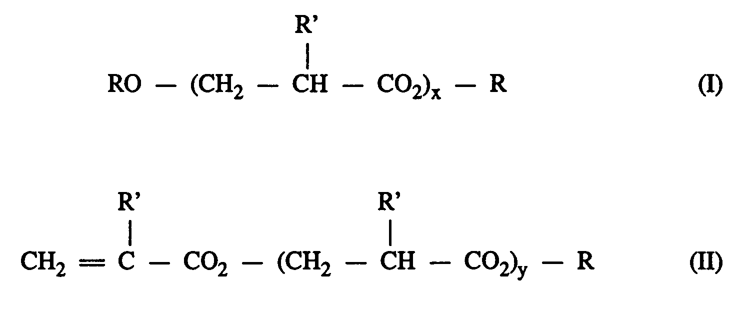 mit
- x,y: = 1-5
- R: = Alkyl
- R': = H oder CH₃

Wem R' = H ist, handelt es sich um eine Veresterung der Acrylsäure, wenn R' = CH₃ ist, handelt es sich um eine Veresterung der Methacrylsäure.

Bei der Herstellung von Estern der Acrylsäure ist das Problem der Oxyesterbildung besonders akut, wobei die hauptsächlich gebildeten Oxyester der Alkoxypropionsäureester und der Acyloxypropionsäureester mit x,y = 1 sind. Bei der Herstellung von Estern der Methacrylsäure erfolgt die Oxyesterbildung in geringerem Maße. Die Entstehung von Oxyestern ist in der DE-A 23 39 529 beschrieben. Aus dieser geht hervor, daß die Bildung von Oxyestern im wesentlichen unabhängig von den speziellen Veresterungsbindungen erfolgt. Von ganz besonderer Bedeutung ist die Oxyesterbildung bei der Herstellung von Acrylaten der C₁- bis C₈-Alkanole, insbesondere der C₄- bis C₈-Alkanole, ganz besonders bei der Herstellung von n-Butylacrylat und 2-Ethylhexylacrylat.

Charakteristisch für die Oxyester ist, daß ihr Siedepunkt oberhalb der Siedepunkte von Ausgangssäure, Ausgangsalkohol, gebildetem Zielester sowie gegebenenfalls mitverwendetem organischem Lösungsmittel liegt.

Die Aufarbeitung eines beliebigen Veresterungs-Reaktionsgemisches erfolgt normalerweise so, daß nicht umgesetzte Ausgangsverbindungen sowie der Zielester vom Reaktionsgemisch destillativ abgetrennt werden, wobei der zur Veresterung verwendete Säurekatalysator gegebenenfalls vorher durch Extraktion mittels Wasser und/oder wäßriger Lauge abgetrennt wird (vgl. z.B. Ullmann's Encyclopedia of Industrial Chemistry, Vol. A1, 5th Ed., VCH, S. 167ff). Das im Rahmen einer solchen destillativen Aufarbeitung verbleibende Sumpfprodukt enthält die Oxyester, die einen beträchtlichen Ausbeuteverlust bedingen.

Es sind daher weitere verschiedenartige Verfahren untersucht worden, um die durch das Auftreten der Oxyester entstehenden Probleme zu lösen. So beschreibt die JP-A-82/62229 die alkalische Verseifung des hochsiedenden Veresterungsrückstandes. Man erhält auf diese Weise einen Teil des eingesetzten Alkohols und Acrylsäure und β-Hydroxypropionsäure bzw. deren Salze zurück. Eine einfache und wirtschaftliche Rückführung der Produkte in die Veresterungsreaktion ist daher nicht möglich. Die JP-AS-72/15936 beschreibt die Gewinnung von Acrylestern durch Umsetzung von β-Alkoxypropionsäureestern mit Acrylsäure in Gegenwart von starken Säuren (Umesterung). Als Nebenprodukt fallen dabei jedoch äquimolare Mengen an β-Alkoxypropionsäure an, die nicht in die Veresterungsreaktion zurückgeführt werden können und daher Abfall darstellen. Die JP-A-93/25086 beschreibt die Spaltung des Michael-Additionsproduktes β-Butoxypropionsäurebutylesters (s. Formel I, x = 1, R = Butyl) bei erhöhter Temperatur und in Gegenwart von Schwefelsäure und eines Überschusses an Wasser. Der Umsatz beträgt jedoch lediglich ca. 30 %. Schließlich beschreibt JP-A-94/65149 die Spaltung der Michael-Additionsprodukte I und II (s.o. x = y = 1) in Gegenwart von Titanalkoholaten. Hier ist der Umsatz ebenfalls gering (< 60%) und es sind hohe Titanatmengen notwendig. Daher ist dieses Verfahren unwirtschaftlich und wegen der großen zu entsorgenden Titanatmengen umweltbelastend.

In der GB 923 595 ist die Gewinnung von Monomeren aus dem Rückstand der Veresterung von Acrylsäure mit Alkanolen in Abwesenheit von molekularem Sauerstoff beschrieben. Empfohlen wird u.a. die Entfernung von allen flüchtigen Monomeren vor der Spaltung, die Spaltung in Gegenwart von Schwefelsäure und die Entfernung der Spaltprodukte mit Hilfe eines Inertgasstromes. Entsprechend den Ausführungsbeispielen wird die Spaltung immer bei min. 300 °C durchgeführt. Als Rückstand entsteht Koks (17-40%). Dieser muß "bergmännisch" aus dem Reaktor entfernt werden. Daher ist dieses Verfahren weder wirtschaftlich noch im technischen Maßstab durchführbar. Ein weiterer Nachteil ist der notwendige Sauerstoffausschluß.

Die CN-A 1,063,678 beschreibt die Spaltung des im Veresterungsrückstand enthaltenen Alkoxypropionsäureesters in Gegenwart von Schwefelsäure in einer Kaskade, wobei Temperatur und Katalysatorkonzentration (0,8 - 1,5 %) in jedem Reaktor unterschiedlich sind. Gekoppelt mit der Spaltung ist eine Destillation zur Trennung von Alkanol und Acrylat. Das Verfahren ist sehr umständlich und erreicht keine hohen Umsätze.

Schließlich beschreibt die CN-A 105 8390 die Spaltung von Alkoxypropionsäureestern in Gegenwart von Schwefelsäure etc. in Alkanole und Acrylsäureester. Es wird dabei schrittweise vorgegangen. Zuerst wird die Spaltung unter Rückfluß durchgeführt und anschließend die Reaktionsprodukte abdestilliert. Die Spaltung der acrylsäurehaltigen Esterrückstande der Ethyl/Methylacrylatherstellung (Ethyl-ethoxypropionat, Methyl-methoxypropionat) wird in Gegenwart von Ethanol bzw. Methanol durchgeführt. Auch hier ist das Verfahren kompliziert und erreicht keine hohen Umsätze.

Der Erfindung liegt die Aufgabe zugrunde, die Rückspaltung der in diesem Sumpfprodukt enthaltenen Oxyester und die Weiterverwendung der dabei anfallenden Ausgangssäure, Ausgangsalkohol und Zielester im Rahmen der Veresterung ohne die Nachteile der Verfahren nach dem Stand der Technik durchzuführen.

Diese Aufgabe wird erfindungsgemäß dadurch gelöst, daß zunächst das Sumpfprodukt abgetrennt, danach die in ihm enthaltenen Oxyester destillativ abgetrennt und das dabei anfallende Destillat in Anwesenheit von Säuren bei erhöhter Temperatur gespalten wird. Die Menge der abdestillierten Oxyester beläuft sich in der Regel auf 75 bis 95 Gew.-% des Sumpfproduktes. Gemäß einer vorteilhaften Ausbildung der Erfindung wird das Verfahren im Beisein von molekularem Sauerstoff durchgeführt.

Es wurde auch schon vorgeschlagen, die Rückspaltung der Oxyester im Sumpfprodukt befindlich durchzuführen, diese Verfahrensweise ist jedoch mit dem Nachteil behaftet, daß nach beendeter Rückspaltung ein hochviskoser Rückstand verbleibt, der schwierig zu entsorgen ist. Überraschenderweise weist das erfindungsgemäße Verfahren diesen Nachteil nicht auf. Außerdem vermag bei einer halbkontinuierlichen Ausführungsweise des erfindungsgemäßen Verfahrens eine vorgelegte Menge sauren Spaltkatalysators mehr an kontinuierlich zugeführtem Edukt zu spalten als im Falle einer im Sumpfprodukt durchgeführte Spaltung.
Sowohl der beim Abdestillieren der Oxyester verbleibende Rückstand als auch der Rückstand des Spaltverfahrens sind leichtflüssig.

Gemäß einer vorteilhaften Weiterbildung der Erfindung werden dem Destillat als Säuren beispielsweise Mineralsäuren, wie Schwefelsäure oder Phosphorsäure, und/oder organische Säuren wie Alkyl- oder Arylsulfonsäuren, beispielsweise Methansulfonsäure oder p-Toluolsulfonsäure, zugesetzt. Dabei kann die dann insgesamt enthaltene Säuremenge 1 bis 20 Gew.-%, vorzugsweise 5 bis 15 Gew.-% bezogen auf die Mengen des Sumpfprodukts, betragen. Besonders günstig ist es, wenn als Schleppmittel für die Spaltprodukte ein Strippgas, das vorzugsweise molekularen Sauerstoff enthält, durch das Sumpfprodukt geführt wird. Zweckmäßigerweise werden als Strippgas Luft oder Gemische von Luft mit Inertgas (z.B. Stickstoff) verwendet.

Für die Aufarbeitung der bei der Veresterung als Sumpfprodukt anfallenden Oxyester kann ein einfacher beheizbarer Rührreaktor mit Doppelwandheizung oder Heizschlange, oder auch ein Zwangsumlaufverdampfer, beispielsweise ein Fallfilmverdampfer oder Flashverdampfer, gekoppelt mit einem Verweilzeitbehälter verwendet werden. Zur besseren Trennung der Spaltprodukte ist ggf. eine auf die Spaltapparatur aufgesetzte Rektifikationsvorrichtung, z.B. eine Füllkörper-, Packungs- oder Bodenkolonne zweckmäßig. Diese Rektifikationsvorrichtung wird in der Regel mit Polymerisationsinhibitoren (z.B. Phenothiazin, Hydrochinonmonomethylether etc.) stabilisiert betrieben.

Die Vorteile des erfindungsgemäßen Verfahrens sind vor allem darin zu sehen, daß mit diesem höhere Umsätze erzielbar sind, als mit bekannten Verfahren. Zusätzlich von Vorteil ist, daß kein Verdünnungsmittel notwendig ist. Außerdem sind geringere Katalysatormengen erforderlich und es ergibt sich eine geringere Umweltbelastung, da geringere Mengen an Rückstand entsorgt werden müssen.

Die Destillationsbedingungen richten sich nach der Art der bei der Veresterung eingesetzten Alkoholkomponente. In der Regel sind eine Temperatur von 100 bis 300 °C und ein Druck von 1 bis 50 mbar vorgesehen. Für das Verfahren eignet sich jede herkömmliche Destillationsapparatur. Da nur eine einfache Trennaufgabe zu lösen ist, genügt in der Regel ein einfacher Spritzschutz, d.h. eine Kolonne ist normalerweise nicht erforderlich.

Die Bedingungen für die Durchführung des erfindungsgemäßen Verfahrens der Spaltung der Oxyester, die aus dem Sumpfprodukt abdestilliert wurden, sind folgende:
- Katalysator:: wenigstens eine Säure aus der Gruppe umfassend Mineralsäuren, wie z.B. Schwefelsäure und Phosphorsäure, sowie organische Säure, wie z.B. Alkyl- oder Arylsulfonsäuren, beispielsweise Methansulfonsäure oder p-Toluolsulfonsäure
- Katalysatormenge:: 1 - 20 Gew.-%, vorzugsweise 5 - 15 Gew.- % bezogen auf die Menge des Oxyesterdestillats
- Temperatur:: 150 - 250 °C, vorzugsweise 180 - 230 °C
- Druck:: vorzugsweise drucklos bzw. reduzierter Druck (so daß die Spaltprodukte unmittelbar abdampfen)
- ggf. Strippgas Menge:: 10 - 100 l/h l
- Reaktionszeit:: 1 - 10 Std.
- Umsatz:: ≥ 90 %

Die Reaktionsführung läuft z.B. in der Weise, daß das zu spaltende Oxyesterdestillat aus dem Sumpfprodukt, kontinuierlich mit dem Spaltkatalysator dem Spaltreaktor zugeführt wird. Die Reaktionsführung kann auch diskontinuierlich, d.h. batchweise, durchgeführt werden. Auch eine halbkontinuierliche Reaktionsführung ist möglich, bei der das zu spaltende Produkt kontinuierlich dem Spaltreaktor, der den Spaltkatalysator enthält, zugeführt wird der verbleibende Sumpf erst nach Beendigung der Spaltung aus dem Spaltreaktor batchweise entfernt wird. Die Spaltprodukte werden kontinuierlich destillativ abgetrennt. Wie bereits ausgeführt kann es vorteilhaft sein, die Spaltung in Gegenwart eines Strippgases (z.B. Luft) durchzuführen. Auf diese Weise werden die Spaltprodukte rasch aus dem Reaktionsgemisch entfernt und die Bildung unerwünschter Nebenprodukte reduziert.

Die Anwendbarkeit des beschriebenen Spaltverfahrens ist nicht auf eine spezielle Natur des Veresterungsprozesses, als dessen Nebenprodukte die Oxyester, also die Additionsverbindungen I und II, anfallen, beschränkt. In der Regel werden die Ester nach den üblichen Verfahren hergestellt (s. Ullmann's Encyclopedia of Industrial Chemistry, Vol. A1, 5th Ed., VCH, S. 167ff).

Ein typisches Beispiel für die Bedingungen, unter denen die Veresterung stattfinden kann, läßt sich kurz wie folgt darstellen:
- Alkohol: (Meth)acrylsäure 1 :: 0,7 - 1,2 (molar)
- Katalysator:: Schwefelsäure oder Sulfonsäuren
- Katalysatormenge:: 0,1 - 10 Gew.-% (vorzugsweise 0,5 - 5%) bzgl. Einsatzstoffe
- Stabilisierung:: 200 - 2000 ppm Phenothiazin (bezogen auf das Gewicht der Einsatzstoffe)
- Reaktionstemperatur:: 80 - 160 °C, vorzugsweise 90 - 130 °C
- Reaktionszeit:: 1 - 10 Std., vorzugsweise 1 - 6 Std.

Gegebenenfalls wird ein Schleppmittel (z.B. Cyclohexan oder Toluol) zur Entfernung des Veresterungswassers eingesetzt. Die Veresterung kann drucklos, mit Überdruck oder Unterdruck sowohl kontinuierlich als auch diskontinuierlich durchgeführt werden.

Bei der säurekatalysierten Veresterung von Acrylsäure mit Alkanolen hat das nach der Abtrennung des sauren Veresterungskatalysators, der nicht umgesetzten Ausgangsstoffe und des Acrylesters resultierende Sumpfprodukt in der Regel folgende Zusammensetzung:
- 1 - 20 Gew.- %: Acrylester
- 50 - 80 Gew.-%: Alkoxypropionate (s. Formel I)
- 5 - 30 Gew.-%: Acyloxypropionate (s. Formel II)
Rest: hauptsächlich Stabilisatoren (Phenothiazin) und Polymere

Weitere Einzelheiten und Vorteile des erfindungsgemäßen Verfahrens können den im folgenden beschriebenen Ausführungsbeispielen entnommen werden.

### Vergleichsbeispiel

Ein aus Glas bestehender Umlaufreaktor (Volumen: 1 l), beheizt mit einer Heizkerze, wurde mit 40 g p-Toluolsulfonsäure und 500 g eines vom sauren Veresterungskatalysator befreiten Veresterungsrückstandes der Butylacrylatherstellung gefüllt. Im folgenden steht Butyl für n-Butyl. Der Rückstand enthielt 10,1 Gew.-% Butylacrylat, 65,4 Gew.-% Butoxyester I und 20,0 Gew.-% Acyloxyester II (R = C₄H₉). Der Rest bestand aus Polymeren, Oligomeren und Polymerisationsinhibitor (Phenothiazin). Die Spalttemperatur betrug 195 °C und der Arbeitsdruck lag bei 1 atm.

Standgeregelt wurde dem Spaltreaktor während der Spaltung kontinuierlich Veresterungsrückstand zugeführt.

Die Spaltprodukte wurden dampfförmig abgeführt und kondensiert. Zwischen Reaktor und Kondensator befand sich eine leere Kolonne (50 cm x 2,8 cm) als Spritzschutz. Innerhalb von 21,5 Stunden konnten auf diese Weise 1589 g Veresterungsrückstand der Spaltung zugeführt werden. Entsprechend der gaschromatographischen Analyse enthielt das anfallende Kondensat (1278 g):
- 69,1 Gew.-%: Butylacrylat
- 18,3 Gew.-%: Butanol
- 6,5 Gew.-%: Acrylsäure
- 7,0 Gew.-%: Olefine und Ether
- 3,5 Gew.-%: Butoxypropionsäurebutylester
Umsatz: 84 Gew.-% bezogen auf Oxyester.

Der Spaltrückstand war bei Raumtemperatur zäh und enthielt Feststoffe. Erst nach Zugabe eines üblichen Lösungsmittels war der Rückstand pumpfähig.

Die folgenden Beispiele zeigen die mit dem erfindungsgemäßen Verfahren erzielten Ergebnisse. Diese Beispiele sind unterteilt in die Verfahrensabschnitte:
**A** - Destillation der bei der Veresterung anfallenden Sumpfflüssigkeit
**B** - Spaltung des gemäß A entstehenden Destillats

### Beispiel 1

### 1A-Destillation

Eine Destillationsapparatur, bestehend aus einem Rundkolben (21), einer aufgesetzten Kolonne (50 cm x 2,8 cm; Raschigringe 5 mm) und einem Kondensator, wurde mit 1 l eines bei der Herstellung von Butylacrylat anfallenden, keinen sauren Veresterungskatalysator mehr aufweisenden Sumpfflüssigkeit folgender Zusammensetzung gefüllt:
- 10,1 Gew.-%: Butylacrylat
- 65,4 Gew.-%: Butoxyester I (R = C₄H₉)
- 20,0 Gew.-%: Acyloxyester II (R = C₄H₉)
Rest: hauptsächlich Polymere und Phenothiazin (Polymerisationsinhibitor)

Die Destillationstemperatur betrug 145 °C, der Druck 30 mbar. Der Flüssigkeitsstand im Destillationskolben wurde durch kontinuierliche Zugabe der Sumpfflüssigkeit (300 g/h) konstant gehalten. 10 Gew.-% der Zulaufmenge wurden als Destillationssumpf aus der Destillationsapparatur ausgeschleust. Das anfallende Destillat enthielt entsprechend der gaschromatographischen Analyse:
- 11,0 Gew.-%: Butylacrylat
- 64,8 Gew.-%: Butoxyester I (R = C₄H₉)
- 20,5 Gew.-%: Acyloxyester II (R = C₄H₉)

Eine Stabilisierung der Kolonne mit Phenothiazin oder einer anderen üblichen Stabilisierung war nicht notwendig. Der anfallende Destillationssumpf war bei 25 °C noch gut handhabbar (pumpfähig) und enthielt keine Feststoffe.

### 1B-Spaltung

Ein aus Glas bestehender Umlaufreaktor (Volumen 1 l), beheizt mit einer Heizkerze, wurde mit 500 g des Destillats der Destillation des Veresterungsrückstandes (1A) und 40 g p-Toluolsulfonsäure gefüllt. Die Spalttemperatur betrug 195 °C. Der Arbeitsdruck lag bei 1 atm. Standgeregelt wurde dem Spaltreaktor das zu spaltende Gemisch kontinuierlich zugeführt. Die Spaltprodukte wurden dampfförmig abgeführt und am Kopf der auf dem Spaltreaktor aufgesetzte Kolonne (50 cm x 2,8 cm, leer) kondensiert. Innerhalb von 119,5 Stunden konnten 7401 g Gemisch der Spaltung zugeführt und 7080 g Spaltprodukte kondensiert werden. Entsprechend der gaschromatographischen Analyse enthielt das Kondensat
- 72,0 Gew.-%: Butylacrylat
- 13,9 Gew.-%: Butanol,
- 4,8 Gew.-%: Acrylsäure,
- 1,4 Gew.-%: Dibutylether
- 6,6 Gew.-%: Butene
- 0,2 Gew.-%: Butoxypropionsäurebutylester
Umsatz: 96 Gew.-%

Das Sumpfprodukt der Spaltung war bei 25 °C noch gut handhabbar (pumpfähig) und enthielt keine Feststoffe.

### Beispiel 2

### 2A-Destillation

Eine Destillationsapparatur, bestehend aus einem 21 Rundkolben, einer aufgesetzten Kolonne (30 cm x 2,8 cm, 5 mm Raschigringe) und einem Kondensator, wurde mit 1000 g eines bei der Herstellung von 2-Ethylhexylacrylat anfallenden, keinen sauren Veresterungskatalysator mehr aufweisende Sumpfflüssigkeit folgender Zusammensetzung gefüllt:
- 65,0 Gew.-%: Alkoxyester I (R = C₈H₁₇)
- 5,5 Gew.-%: Acyloxyester II (R = C₈H₁₇)
- 2,1 Gew.-%: 2-Ethylhexylacrylat
- 1,0 Gew.-%: Di-2-ehtylhexylether
Rest: Polymere, Oligomere, Polymerisationsinhibitor (Phenothiazin)

Die Destillation wurde bei 1 mbar bis zu einer Sumpftemperatur von 250 °C durchgeführt. Das Destillat (763 g) enthielt entsprechend der gaschromatographischen Analyse:
- 89,5 Gew.-%: Alkoxyester I (R = C₈H₁₇)
- 5,2 Gew.-%: Acyloxyester II (R = C₈H₁₇)
- 3,5 Gew.-%: 2-Ethylhexylacrylat
- 1,0 Gew.-%: Di-2-ethylhexylether

Der anfallende Destillationssumpf war bei 25 °C noch gut handhabbar (pumpfähig) und enthielt keine Feststoffe.

### 2B-Spaltung

Ein Spaltreaktor bestehend aus einem 1 l Rührreaktor, einer aufgesetzten Kolonne (30 cm x 2,8 cm, 5 mm Raschigringe) und Kondensator wurden mit 500 g Destillat aus der Destillation (2A) und 10 g p-Toluolsulfonsäure gefüllt. Die Spaltung wurde bei 180 °C und 50 mbar Druck durchgeführt. Reaktionszeit: 2 Stunden. Das Kondensat (570 g) enthielt entsprechend der gaschromatographischen Analyse:
- 1,4 Gew.-%: Acrylsäure
- 16,2 Gew.-%: 2-Ethylhexanol
- 70,9 Gew.- %: 2-Ethylhexylacrylat
- 3,7 Gew.-%: Di-2-ethylhexylether
- 6,1 Gew.-%: Octene
- 1,8 Gew.- %: Alkoxyester I (R = C₈H₁₇)
Umsatz: 95 Gew.-%

Das Sumpfprodukt der Spaltung war bei 25 °C gut handhabbar (pumpfähig) und enthielt keine Feststoffe.

Den vorstehenden Beispielen des erfindungsgemäßen Verfahren ist zu entnehmen, daß mit diesem Verfahren einerseits erheblich höhere Umsätze erzielbar sind als mit bekannten Verfahren, und daß keine Verdünnungsmittel erforderlich sind, um das bei der Spaltung anfallende Sumpfprodukt zu entfernen.

## Patentansprüche

1. Verfahren zum Verestern von (Meth)acrylsäure mit einem Alkanol in Gegenwart eines Veresterungskatalysators, bei dem nicht umgesetzte Ausgangsverbindungen und der zu bildende (Meth)acrylsäureester destillativ abgetrennt werden und ein Oxyester enthaltendes Sumpfprodukt entsteht, **dadurch gekennzeichnet, daß** zunächst das Sumpfprodukt abgetrennt, danach die in ihm enthaltenen Oxyester destillativ abgetrennt und das dabei anfallende Destillat in Anwesenheit von Säuren bei erhöhter Temperatur gespalten wird.

2. Verfahren nach Anspruch 1, dadurch gekennzeichnet, daß das Verfahren im Beisein von molekularem Sauerstoff durchgeführt wird.

3. Verfahren nach Anspruch 1 oder 2, dadurch gekennzeichnet, daß das Destillat bei Temperaturen von 170 bis 250 °C, vorzugsweise bei 180 bis 230 °C gespalten wird.

4. Verfahren nach Anspruch 1, 2 oder 3, dadurch gekennzeichnet, daß dem Destillat als Säure eine solche aus der Gruppe umfassend Mineralsäuren wie Schwefelsäure oder Phosphorsäure, und organische Säuren wie Alkyl- oder Arylsulfonsäuren, z.B. Methansulfonsäure oder p-Toluolsulfonsäure, zugesetzt wird.

5. Verfahren nach Anspruch 4, dadurch gekennzeichnet, daß die zugesetzte Säuremenge 1 bis 20 Gew.-%, vorzugsweise 5 bis 15 Gew.-%, bezogen auf das Destillat, beträgt.

6. Verfahren nach einem der Ansprüche 1 bis 5, dadurch gekennzeichnet, daß die Spaltung bei reduziertem Druck (< 1 atm) durchgeführt wird.

7. Verfahren nach einem der Ansprüche 1 bis 6, dadurch gekennzeichnet, daß zur Entfernung der Spaltprodukte ein Strippgas durch das zu spaltende Destillat geführt wird.

8. Verfahren nach Anspruch 7, dadurch gekennzeichnet, daß als Strippgas ein Sauerstoff enthaltendes Gas verwendet wird.

9. Verfahren nach einem der vorhergehenden Ansprüche, dadurch gekennzeichnet, daß die anfallenden Spaltprodukte unmittelbar in die Veresterung rückgeführt werden.

10. Verfahren nach einem der vorhergehenden Ansprüche, dadurch gekennzeichnet, daß das Destillat aus dem Sumpfprodukt der Veresterung mit n-Butanol oder 2-Ethylhexanol gewonnen wurde.

## Claims

1. A process for esterifying (meth)acrylic acid with an alkanol in the presence of an esterification catalyst, in which unconverted starting compounds and the (meth)-acrylate to be formed are separated off by distillation and an oxyester-containing bottom product is formed, wherein the bottom product is first separated off, the oxyesters contained therein are then separated off by distillation and the resulting distillate is cleaved in the presence of acids at elevated temperatures.

2. A process as claimed in claim 1, which is carried out in the presence of molecular oxygen.

3. A process as claimed in claim 1 or 2, wherein the distillate is cleaved at from 170 to 250°C, preferably from 180 to 230°C.

4. A process as claimed in claim 1, 2 or 3, wherein the acid added to the distillate is one selected from the group consisting of mineral acids, such as sulfuric acid and phosphoric acid, and organic acids, such as alkanesulfonic and arylsulfonic acids, eg. methanesulfonic acid or p-toluenesulfonic acid.

5. A process as claimed in claim 4, wherein the amount of acid added is from 1 to 20, preferably from 5 to 15, % by weight, based on the distillate.

6. A process as claimed in any of claims 1 to 5, wherein the cleavage is carried out at reduced pressure (< 1 atm).

7. A process as claimed in any of claims 1 to 6, wherein, in order to remove the cleavage products, a stripping gas is fed through the distillate to be cleaved.

8. A process as claimed in claim 7, wherein the stripping gas used is an oxygen-containing gas.

9. A process as claimed in any of the preceding claims, wherein the cleavage products obtained are recycled directly to the esterification.

10. A process as claimed in any of the preceding claims, wherein the distillate was obtained from the bottom product of the esterification with n-butanol or 2-ethylhexanol.

## Revendications

1. Procédé d'estérification d'acide acrylique ou méthacrylique par un alcanol en présence d'un catalyseur d'estérification, dans lequel les composés de départ qui n'ont pas réagi et l'ester d'acide acrylique ou méthacrylique à former sont isolés par distillation et un produit de fond de colonne contenant de l'oxyester est formé, caractérisé en ce qu'on isole tout d'abord le produit de fond de colonne, puis on isole par distillation l'oxyester contenu dans celui-ci et on scinde le distillat formé en présence d'acides à une température élevée.

2. Procédé suivant la revendication 1, caractérisé en ce que le procédé est effectué en présence d'oxygène moléculaire.

3. Procédé suivant l'une des revendications 1 et 2, caractérisé en ce que le distillat est scindé à des températures de 170 à 250°C, de préférence à 180 jusqu'à 230°C.

4. Procédé suivant l'une des revendications 1 à 3, caractérisé en ce qu'on ajoute au distillat, comme acide, un acide du groupe comprenant des acides minéraux, tels que de l'acide sulfurique ou de l'acide phosphorique, et des acides organiques, tels que des acides alkylsulfoniques ou arylsulfoniques, par exemple de l'acide méthanesulfonique ou de l'acide p-toluènesulfonique.

5. Procédé suivant la revendication 4, caractérisé en ce que la quantité d'acide ajoutée est de 1 à 20% en poids, de préférence de 5 à 15% en poids, par rapport au distillat.

6. Procédé suivant l'une des revendications 1 à 5, caractérisé en ce que la scission est effectuée à une pression réduite (< 1 atmosphère).

7. Procédé suivant l'une des revendications 1 à 6, caractérisé en ce qu'un gaz de stripage est conduit à travers le distillat à scinder pour l'élimination des produits de scission.

8. Procédé suivant la revendication 7, caractérisé en ce que, comme gaz de stripage, on utilise un gaz contenant de l'oxygène.

9. Procédé suivant l'une des revendications précédentes, caractérisé en ce que les produits de scission obtenus sont directement recyclés dans l'estérification.

10. Procédé suivant l'une des revendications précédentes, caractérisé en ce que le distillat est obtenu à partir du produit de fond de colonne de l'estérification avec du n-butanol ou du 2-éthylhexanol.
